# EUROPEAN PATENT APPLICATION

(11) **EP 4 825 320 A1**
(43) Date of publication of application: **21.10.2026**
(21) Application number: 25171078.6
(22) Date of filing: 16.04.2025
(51) Int. Cl.: A61B 6/02, A61B 6/03, A61B 6/04, A61B 6/40, A61B 6/00, A61B 6/42

(54) **STEREOSCOPIC IMAGING SYSTEM**

(71) Applicant: Siemens Healthineers International AG, 6312 Steinhausen (CH)
(72) Inventor: Filiberti, Reto, 6340 Baar (CH); Morf, Daniel, 8414 Buch am Irchel (CH)
(74) Representative: Müller Schupfner & Partner Patent- und Rechtsanwaltspartnerschaft mbB (Muc)

(57) **Abstract**

The invention relates to an imaging system for imaging an area of interest of an object (50), comprising a first imaging X-ray source (30), a second imaging X-ray source (40), at least one imager (10), and a moveable support (20), in particular a patient couch, for supporting the object (50), wherein the first and second imaging X-ray sources are configured to irradiate overlapping fields of view within the area of interest, such that projection images acquired by the at least one imager (10) result in a stereoscopic view in a region where the fields of view overlap, and wherein the first imaging X-ray source (30), the second imaging X-ray source (40) and the support (20) are configured to move in synchrony with each other.

## Description

The present invention relates to an imaging system, in particular for motion supervision of a patient undergoing radiation treatment, a radiation treatment system and a use of an imaging system.

Independent of the grammatical term usage, individuals with male, female or other gender identities are included within the term.

X-ray imaging systems may be used for a variety of applications such as medical-, industrial- or inspections-related applications. One possible medical application is in connection with radiotherapy, where the imaging system is used to detect patient motion during the course of treatment, and to locate a target volume to be treated with radiation.

Radiotherapy involves delivering a prescribed radiation dose to a specific geometrically defined target volume (also referred to as target). Generally, a radiation therapy treatment plan is formed based on the location and orientation of the target volume and surrounding structures in an initial computed tomography (CT) image or magnetic resonance image. Typically, this treatment is delivered to a patient in one or more therapy sessions. In order to reduce the dosage to surrounding healthy structures, the location of the target volume relative to the radiation therapy source must be known precisely in each treatment session and during each treatment session in order to accurately deliver the radiation dose while minimizing complications in healthy structures. Therefore, radiotherapy treatment systems often include imaging systems for intrafraction motion monitoring.

US 2019/0168025 A1, for example, discloses an image-guided radiation therapy system comprising a source of a radiation treatment beam and an X-ray imaging system having two imaging beam sources and two related imagers. While this imaging system is capable of proving stereoscopic view of the target structures, it has the disadvantage that the imaging system is not in a fixed orientation relative to the patient. Thus, when the patient couch is moved (e.g. rotated) to bring the patient in a better position for the radiation treatment beam, this will result in a change of the image orientation. Therefore, the image comparison between the acquired images and the CT-image forming the basis of the treatment plan, which is required to detect unwanted movement of the patient, has to be done on recalculated images which take into account the couch movement. This requires considerable computing time and may result in errors.

Other radiation treatment systems, such as the Varian TrueBeam^{®}, include an imaging system having a single X-ray source and an imager, which are configured to rotate around the patient couch. However, this imaging system is coupled to the treatment gantry and therefore requires to move the treatment gantry to specific positions. Further, instantaneous stereo images cannot be provided by the single imager.

It is therefore an object of the present invention to provide an improved imaging system that overcomes at least some of the above-mentioned problems. In particular, it is an object of the present invention to provide an imaging system, which is compact and at the same time allows to acquire instantaneous three-dimensional spatial information from an area of interest.

This object is achieved by an imaging system according to claim 1, a radiation treatment system according to claim 14 and a use of an imaging system according to claim 15. Preferred embodiments are disclosed in the dependent claims.

According to a first aspect, the present invention relates to an imaging system for imaging an area of interest of an object, that comprises:
- a first imaging X-ray source,
- a second imaging X-ray source,
- at least one imager, and
- a moveable support, in particular a patient couch, for supporting the object,

wherein the first and second imaging X-ray sources are configured to irradiate overlapping fields of view within the area of interest, such that projection images acquired by the at least one imager result in a stereoscopic view in a region where the fields of view overlap, and.
wherein the first imaging X-ray source, the second imaging X-ray source and the support are configured to move in synchrony with each other.

Therefore, when the first imaging X-ray source, the second imaging X-ray source and the support move in synchrony with each other, the view of the object and/or the area of interest of the object positioned on the support from the respective X-ray source can be maintained and is not constantly changing. Thus, the projection images acquired from the object while it is placed on the support, e.g. during a radiation treatment session, can be compared with one another, without the need for a reconstruction, recalculation or correction calculation. In other words, the user always has the identical view of the area of interest, and it is therefore easier to detect any deviation in the image due to any patient related movement. In useful embodiments, the imaging system is mounted on the support and moves with it when the support gets moved or adjusted. It is preferably a compact imaging system. The imaging system according to the present invention is preferably not intended to be part of a system that rotates around the support during measurement, such as for example a gantry system including a treatment radiation source or a C-arm. This reduces the risk of patient collisions.

The object can be a human, for example a patient, an animal, or an item to be inspected. The object can be imaged before or during a medical treatment, for example a radiation treatment, or during a medical intervention.

The object is placed on a support, wherein the support may be a table or other receptacle. For medical applications, the support is preferably a patient couch or chair. The support is moveable in at least one direction, for example the vertical direction, in order to adjust the position of the object. In some embodiments, the support is linearly movable in 1, 2 or 3 directions. In other embodiments, the support is additionally rotatable around one or several rotation axes. Thus, in an embodiment, the support is movable in all six degrees for freedom. Thus, the support and the object can be positioned and moved as best suited for the respective application and/or procedure, for example for radiation therapy. When such movement is performed, the imaging system moves with the support.

Within the object, usually only a part of said object is of interest to be investigated. Said part within the object is called the area of interest. The area of interest can be, for example, an organ, or a part of a body such as the abdomen, the chest, the head, an arm, a hand, a leg or a foot. The area of interest is preferably imaged in order to locate a target within the area of interest. The target may for example be an organ of interest within the patient body, or it may be one or several fiducials attached to the object/patient. When aiming to detect patient motion such as breathing motion, it is possible to observe the movement of such fiducials attached to the patient chest and abdomen. When the imaging system is part of a radiation treatment system, the target may be a target volume to be treated with a radiation treatment beam, e.g. a tumour.

The imaging system comprises a first imaging X-ray source and a second imaging X-ray source. In the following, the "imaging X-ray sources" are also referred to as "X-ray sources". Each of the X-ray sources is configured to irradiate a field of view (FOV). The field of views may be fan-shaped or cone-shaped, as will be explained in more detail below. In an embodiment, the FOV of at least one of the X-ray sources is adjustable, in particular its position within the object can be adjusted. The FOV(s) can for example be adjusted by moving and/or rotating the respective X-ray source, or by adjusting their filters or collimators. Thereby, the X-ray sources can be made relatively light and compact, since their field of view can be relatively small, but can still reach the entire area of interest within the patient by being adjustable. Preferably, each imaging X-ray source is configured to emit X-rays in the kV-range. Preferably, the X-ray sources comprise small, light-weight X-ray tubes, preferably having stationary anodes. The X-ray sources are preferably air-cooled only.

The projection images of the FOVs can be acquired by the at least one imager, which is configured to receive the radiation emitted from at least one of the X-ray sources. The imager is e.g. an X-ray detector adapted to the emission range of the X-ray sources. Preferably, the imager is a two-dimensional (2D) detector. The at least one imager may for example have a small form factor and light weight. It may be formed by a radiation hard panel.

With two X-ray sources each having one field of view (FOV), the X-ray sources can be directed in such a way that the FOV of the one X-ray source and the FOV of the other X-ray source at least partially overlap. As the two X-ray sources are usually positioned at different locations, the view from the one X-ray source and the view of the other X-ray source will differ and provide different information of the area of interest. Due to this different positioning and overlap, a stereoscopic view of a region where the fields of view overlap (the "region of overlap") is achieved. Stereoscopic x-ray imaging is a method for obtaining three-dimensional (3D) spatial information from two-dimensional (2D) projection X-ray images. In particular, the two projection images of the region of overlap may be digitally analysed in order to provide the 3D position of the target.

The first and second first X-ray sources and the support are configured to move in synchrony with each other. This means that the respective X-ray source and the support are configured to make the same movements within the technically feasible limitations, in particular when the support is being adjusted as described above. Thus, when the support moves in one or more directions, the X-ray source moves in the same one or more directions by the same distance. In other words, the X-ray sources are configured to synchronously follow the movement of the support. Preferably, this is achieved by a physical connection between the X-ray sources and the support. Thereby, the orientation and/or view between the respective X-ray source and the support is maintained even when the support is moved by a linear and/or rotational movement.

The synchronous movement can be achieved in particular by mechanically attaching the first and second X-ray sources to the support, or by attaching support and X-ray sources to the same moveable base. In some embodiments, the X-ray sources may be moveably, in particular slidably connected to the support. For example, they may be adjustable/slidable in the longitudinal direction of a patient couch. Thereby, the area of interest may be adjusted, for example in order to be able to image different body regions. However, while images are being acquired, the X-ray sources are preferably fixed in one position with respect to the patient couch. Thus, also in this embodiment, the X-ray sources are configured to move in synchrony with the support.

In some embodiments, the X-ray sources may be detachably mounted on the support. In other words, they can be attached to the support for imaging, and can be removed e.g. for maintenance. In an example, the imaging system as a whole may be detachably mounted on the support. Thus, the imaging system may be portable. It may for example be mountable on the support in different positions in order to image different regions of interest.

In some embodiments, there may be more than two X-ray sources comprised within the imaging system, such as for example, three, four, five or six. Two or more X-ray sources may provide the possibility for an improved 3D view of a region where the at least two fields of view overlap. With more than two X-ray sources the region of overlap can be increased and/or more easily adapted and/or manipulated. Further, if one X-ray source is not functioning properly or is out of order, a stereoscopic view can still be achieved with two of the remaining X-ray sources.

As an alternative, the imaging system may comprise only one imaging X-ray source. Hereby, the area of interest is only irradiated by one imaging X-ray source and, consequently, there is no stereoscopic view. Nevertheless, said imaging X-ray source and the support are configured to move in synchrony with each other and provide the above-mentioned advantages.

In embodiments where the object is a human and the support is a patient couch, the support has a longitudinal direction along the patient length, i.e. extending from head to foot of the patient, and a lateral direction corresponding to the lateral (left-right) direction of the patient, when positioned on the couch in supine position. The direction from anterior to posterior of the patient when positioned on the support in supine position defines the vertical direction.

In an embodiment, two (or more) X-ray sources are substantially positioned beside each other in the lateral direction, in order to provide a stereoscopic view from substantially the same position in the longitudinal direction. Hereby, "substantially the same position" includes a deviation of up to ±10 cm, preferably up to ±5 cm.

In the vertical direction, the position of the two or more X-ray sources is not limited. In a useful embodiment, the X-ray sources are arranged below the object in the vertical direction, for example below the support, or they are integrated into the support. Alternatively, the X-ray sources may be disposed to the lateral sides of the support, e.g. substantially at the same vertical position as a support surface supporting the object/patient. In this embodiment, the at least one imager is preferably disposed above the support. For example, the imager may be held by one or more support arms which are attached to or attachable to the support. Thereby, the object may be placed on the support between imager and X-ray sources.

In an alternative embodiment, the X-ray sources are arranged above the object in the vertical direction. In this embodiment, the at least one imager is preferably disposed below the support in vertical direction, or may be integrated into the support.

The imaging rate is not particularly limited, but the negative effects of the additional radiation dose should be weighted with the positive effects of the additional information, when the imaging system is used for monitoring patient motion. Several irradiations per second are possible, for example 5-20s⁻¹, but depending on the application, one irradiation every 0.5sec to 5min, preferably every 0,5sec to 30sec, more preferred every 1 to 2sec, may be a reasonable compromise between additional information and additional radiation dose.

The imaging system can also be used in industrial- or inspections-related applications, for example, in order to analyse the material composition of the object or to analyse the contents of the object.

Due to the compact nature of the imaging system, it is particularly suitable for applications where only limited space is available or envisaged. The imaging system can also be a portable system and/or can be implemented in different systems, such as radiation treatment systems.

According to an embodiment of the present invention, the at least one imager is configured to move in synchrony with the first imaging X-ray source, the second imaging X-ray source and the support. Thereby, the imaging configuration stays the same, even when the support is moved/adjusted. For the synchronous movement, the same features and advantages apply as stated above for the synchronous movement of the X-ray sources and the support. In particular, the synchronous movement can be achieved by mechanically attaching the at least one imager to the support, or by attaching support and imager to the same moveable base. For example, the imager may be mounted on the support by means of one or more support arms. In useful embodiments, the position of the imager is adjustable to accommodate different field of views or different areas of interest. For example, the support arm may be adjustable. However, once it is adjusted, the position of imager with respect to the support/couch is configured to stay constant while images are being acquired.

According to an embodiment of the present invention, the first imaging X-ray source, the second imaging X-ray source and/or the at least one imager are mounted on the support.

One preferred possibility to achieve a movement in synchrony of at least two elements such as the first imaging X-ray source, the second imaging X-ray source, the at least one imager and/or the support is by mounting one element on another. Hereby, a physical connection between one of the mentioned elements and the support can be established. Such a connection or mounting does not have to be direct but can also be achieved indirectly, e.g. having a further connection element in between the X-ray source or imager and the support, as long as a movement in synchrony can be achieved. Therefore, the present invention is not limited to any specific type of connection. Such a connection or mounting may be detachable, adjustable and/or rotatable. In preferred embodiments, the position of the first imaging X-ray source, the position of the second imaging X-ray source and/or the position of the at least one imager mounted on the support is adjustable in order to adjust the position of the FOVs of the X-ray sources.

In an embodiment, the imager is detachably mounted, whereas the X-ray sources are statically or slidably attached to the patient couch, which is useful in order to provide more space for the patient to mount and dismount the patient couch.

According to an embodiment of the present invention, the first and second imaging X-ray sources are configured to irradiate the same imager, wherein the imaging system in particular comprises only a single imager.

Thereby, the imaging system can be constructed in a compact fashion. The single imager may be configured to be irradiated by both X-ray sources one after the other. Thus, there is a small time delay in the acquisition of the two stereo images required for stereoscopic view, but the time difference is too small to negatively affect the imaging quality. Alternatively, the imaging system may be configured so that the at least two X-ray sources irradiate different areas on the imager. For example, the FOV of the X-ray sources may be adjustable, as explained herein. Thereby, the X-ray tubes may irradiate different, variable sections of the same imager. This provides instantaneous stereoscopic view of the region where the FOVs of the X-ray tubes overlap. In an embodiment, the X-ray tubes may be rotatable to "point" towards different positions on the imager.

The imaging system may comprise more than two X-ray sources as described herein and each X-ray source may irradiate the same imager.

In alternative embodiments, the imaging system may comprise more than one imager. In these embodiments, the imaging system can be more flexible and more reliable, since the further imagers can be used if they are in a more suitable position or if one imager is not working or in need of inspection or repair.

According to an embodiment of the present invention, at least one of the first imaging X-ray source and the second imaging X-ray source is rotatable around its own axis and/or has a rotatable collimation slot, such that the field of view of the at least one imaging X-ray source is rotatable around the axis of the respective imaging X-ray source.

In useful embodiments, at least one of the X-ray sources has an adjustable field of view. For example, the position of the FOV in the region of interest can be varied. Thereby, the position of the region of overlap may be adjusted in order to capture the relevant target volume. This allows for a more adaptable and more flexible imaging system as well as a reduced total radiation dose due to the smaller FOV of the X-ray sources.

The adjustable FOV may be realised by at least one of the X-ray sources being rotatable around its own axis. Preferably, the X-ray source is rotatable about one axis. In useful embodiments, the rotation axis is oriented in the longitudinal direction of the support, so that the output window or FOV of the X-ray source may be pivoted around the longitudinal axis to cover different regions of the object. In other embodiments, the axis may be oriented in the lateral direction. Preferably, all X-ray sources are rotatable, wherein preferably the orientations of the rotation axes are identical. In an embodiment, the at least one X-ray source is rotatable about more than one axis, for example around two or three axes. Alternatively or in addition, at least one X-ray source may have a rotatable collimation slot, where the collimation slot may rotate around said X-ray source.

As a result of any of the mentioned rotations, the orientation of the respective FOV of the X-ray source may be adjusted. Hereby, the position of the region where the FOVs of the X-ray sources overlap can also be adjusted. In this overlap region, stereo vision can be achieved. One advantage thereof is that the overlapping region can be adjusted to the position of the target within the object.

According to an embodiment of the present invention, the support has a longitudinal axis, and the at least one imager is curved around said longitudinal axis.

The longitudinal axis extends in the longitudinal direction, as defined above. Providing a curved imager has the advantage that the imager is both compact and well adapted to capture the X-rays emitted by the X-ray sources. Preferably, the longitudinal axis forming the centre of curvature is disposed substantially centred in the lateral direction. The curvature may be circular. The radius of the curvature is not particularly limited, whereby a radius between 30cm and 180cm, preferably 50cm to 150cm, may be suitable for the geometrical and technical requirements of the imaging system. For example, a gantry, in particular a C-arm gantry, may be surrounding the support requiring a clearance envelope due to the rotation of said gantry or C-arm around the support during use. The measure of curvature of the imager can be adapted to achieve that the imaging system can be positioned within said clearance envelope.

In an embodiment, the radius of curvature of the imager may be variable and/or adaptable. The measure of curvature of the imager may be greater in at least one part of the imager compared to at least one other part of the imager. For example, at least one side part of the imager in the longitudinal direction may have a greater measure of curvature compared to a middle part of the imager. The radius of curvature of the imager may also be adaptable so that the imager can have a different radius of curvature, for example for different measurements. This may make the imaging system even more compact. The radius of curvature of the imager can e.g. be adapted by providing a flexible imager. For example, the imager may be flexible so that at least a part of the imager can change its radius of curvature by means of exerted pressure. The imager may also be curved around one or more axes, which may include one or more axes different than the longitudinal axis.

The at least one imager of the imaging system may be foldable and removable/detachable. Therefore, when the imaging system is used in the context of radiation treatment, the imager can be removed to clear the way for the radiation treatment beam. For example, the imaging system can be used to determine the position of the target volume to be treated after the patient has been positioned on the couch. Subsequently, the imager is removed and the radiation treatment is performed.

In an embodiment, the at least one imager can be a multi-part imager comprising more than one part, preferably two, three or four parts. The parts may each comprise a flat panel. In useful embodiments, the imager parts may be arranged concentrically around the support. In one embodiment, the multi-part imager comprises more than one separate imager, preferably two, three or four imagers. In another embodiment, the multi-part imager is a split imager comprising one imager having several parts. For example, each part is formed by a flat or curved portion, the portions being contiguous. The multi-part imager may be foldable and/or bendable. In particular, the space of such multi-part imager may be reduced when no projection images are being acquired. This makes the imaging system more flexible and more compact. In other embodiments, the size of the one or more imagers can be adapted to the requirements of the imaging. When the imaging system is not in use such one or more imagers can be stored in a more compact way, reducing an imager to be an obstacle and increasing the safety for the people and the imaging system. The size of the imager may for example be between 25cm to 150cm, preferably 50cm to 100cm. (diameter or edge length of a rectangular imager).

According to an embodiment of the present invention, the at least one imager is rotatable and/or movable, preferably on a circle segment, around the object, in particular by a predetermined angle and/or by predetermined steps, such that there are at least two positions of the at least one imager.

In an embodiment, the at least one imager may be movable by a movement in at least one spatial dimension and/or rotatable by a rotational movement in at least one rotation direction. Thereby, the position of the imager may be adjusted to acquire images from variable FOVs, in particular when at least one of the X-ray sources has an adjustable field of view. Thereby, the imaging system becomes more compact. In other words, a smaller imager may be used, which can nonetheless acquire images from a large area of interest. For example, the imaging system may allow to acquire images from an entire axial cross section of a patient. For example, when the support, the object and/or the at least one X-ray source are changing one's position, it may be recommendable to amend the position of the at least one imager. For example, a further system, such as a gantry or C-arm, may be in the vicinity of the imaging system of the present invention and may result in limitations for said imaging system. As the at least one imager is rotatable and/or movable, the imaging system of the present invention can be adapted to situations due to the environment and/or the use requirements of the imaging system.

One preferable movement of the at least one imager is around a circle element around the object. Hereby, it can be achieved that the distance from the support to the at least one imager is substantially maintained, for example with a variation of ±3 cm, while the position around said circle element can be adapted. Such an adaptation may be necessary or helpful to acquire projection images from different FOVs. Furthermore, it can be achieved that the imaging system remains in a clearance envelope of a surrounding system.

A rotation and/or movement of the at least one imager can be implemented by providing a number of predetermined positions or angles and/or by predetermined steps. Thereby, the at least one imager can be moved and/or rotated using a mechanically robust construction, and the calibration of the imaging system is facilitated.

There may be two possible positions for the at least one imager. Alternatively, there may be more than two possible positions for the at least one imager, preferably, three to ten, more preferably, four to six. This further improves the flexibility and adaptability of the imaging system.

Alternatively, the rotation and/or movement of the at least one imager may be continuous and/or the position may be continuously adjustable. Thereby, a continuous individual positioning can be achieved. A continuous rotation and/or movement of the at least one imager increases the flexibility of the imaging system.

According to an embodiment of the present invention, the at least one imager is positioned above the support and the first imaging X-ray source and the second imaging X-ray source are positioned below the support and/or are integrated into the support.

As the at least one imager is a rather light element, its structural positioning is preferably achieved by a simple and light construction. Therefore, it is a preferred option to place the at least one imager in an area above the support. Consequently, the heavier X-ray sources may preferably be positioned below the support. Alternatively, or in addition at least one of the X-ray sources may be integrated at least partially into the support.

Alternatively, the first imaging X-ray source and the second imaging X-ray source may be disposed to the side of the support, or to the side and below the support. In another alternative, the imager may be positioned below the support and/or may be integrated into the support. Alternatively, the first imaging X-ray source and the second imaging X-ray source may be disposed above the support and the imager may be disposed below the support and/or may be integrated into the support.

According to an embodiment of the present invention, the first imaging X-ray source and/or the second imaging X-ray source are movable relative to the support, in particular along a longitudinal axis of the support.

While the X-ray sources and the support are configured to move in synchrony with each other as stated above, the first imaging X-ray source and/or the second imaging X-ray source may in addition also be movable relative to the support, in order to increase the flexibility and adaptivity of the imaging system. Such a movement may be possible in one or more directions. For example, it may be useful to increase the distance between an imaging X-ray source and the support, for example to increase the FOV of the imaging X-ray source. For example, it may be useful to move an imaging X-ray source in the longitudinal and/or lateral direction, for example to amend the FOV for different measurements and/or for different objects. Alternatively, or in addition it may be useful to rotate the first imaging X-ray source and/or the second imaging X-ray source relative to the support, as disclosed above.

A rotation and/or movement of the first imaging X-ray source, the second imaging X-ray source and/or the support can be implemented by a predetermined angle and/or by predetermined steps. It is mechanically advantageous to provide a construction with such predetermined settings, while providing a flexible and adaptable imaging system. There may be at least two positions of the first imaging X-ray source, the second imaging X-ray source and/or the support. There may be more than two positions of the at least one imager, preferably, three to ten, more preferably, four to six.

Alternatively, the rotation and/or movement of the first imaging X-ray source, the second imaging X-ray source and/or the support may be continuous and the position may be continuously adjustable. Thereby, an individual positioning can be achieved. A continuous rotation and/or movement of the first imaging X-ray source, the second imaging X-ray source and/or the support may increase the flexibility of the imaging system. A movement and/or rotation of the first imaging X-ray source, the second imaging X-ray source and/or the support may reduce the complexity in calibration and/or correction.

The rotation and/or movement of the first imaging X-ray source, the second imaging X-ray source is preferably correlated to the rotation and/or movement of the at least one imager.

According to an embodiment of the present invention, the first imaging X-ray source and/or the second imaging X-ray source are configured to provide a cone beam.

Generally, the imaging system of the present invention can be used by providing a cone beam and/or a fan beam. By using a cone beam, a 2D projection image can acquired instantaneously. This is the fastest way of acquiring images of the field of view. The cone beam may for example have a cone beam angle of 8° to 20° in one direction, and of 20° to 40° in the other direction. To reduce scattering, X-ray sources having relatively narrow cone beams are preferred.

Alternatively, it is possible to use X-ray tubes which provide a fan beam. In this embodiment, the fan beam of each X-ray tube is rotated during image acquisition to sweep the area of interest. Thereby, images may be acquired at a smaller time resolution. However, the radiation scattering is generally lower when using fan beams.

According to an embodiment of the present invention, the at least one imager comprises a scatter rejection grid, in particular a curved scatter rejection grid.

In order to reduce scatter and improve image quality, a scatter rejection grid may be used within at least one part of the at least one imager. In particular, within a curved imager, a curved scatter rejection grid may be used. A scatter rejection grid may comprise lamellae, which are arranged in a grid-like fashion on the imager. A curved scatter rejection grid can be manufactured by additive manufacturing.

According to an embodiment of the present invention, the first imaging X-ray source and/or the second imaging X-ray source are configured to emit multiple X-ray energies.

It is possible to implement imaging X-ray sources that can emit more than one X-ray energy into the imaging system. The X-ray sources may emit X-rays at variable energies, or at different predetermined energies. The use of multiple X-ray energies improves the visibility of soft tissues and can provide material density information. Therefore, using different energies within an imaging system may simplify and/or improve the detection of the target within the area of interest.

According to an embodiment of the present invention, the at least one imager comprises at least one energy discrimination multi-layer imager with at least two layers.

Thereby, one imager can acquire images of different energies. For example, each X-ray source can emit multiple X-ray energies, where the different energies of each X-ray source can be detected by the one energy discrimination multi-layer imager. Alternatively, the more than one X-ray sources can each emit a different energy, where the energy of each X-ray source can be detected by the one energy discrimination multi-layer imager.

According to an embodiment of the present invention, the imaging system comprises more than two, preferably between three and six, more preferred three, four or six, imaging X-ray sources.

Two or more imaging X-ray sources may provide the possibility for a stereoscopic view of a region where at least two fields of view overlap. With more than two X-ray sources the region of overlap can be more easily adapted and/or manipulated and/or a larger region of overlap can be achieved. Further, if one X-ray source is not functioning properly or is out of order, a stereoscopic view can still be achieved with two other X-ray sources.

Two X-ray sources can be substantially positioned beside each other in the lateral direction to provide a stereoscopic view of an overlapping region from substantially the same position in the longitudinal direction. Hereby, substantially the same position includes a deviation in the longitudinal direction of up to 10 cm. In the vertical direction the position of the two X-ray sources is not limited. If more than two X-ray sources are present, the further X-ray sources may also be present at substantially the same position in the longitudinal direction. This could improve and/or increase the stereoscopic view as there can be an overlap of three, four or even more fields of view and/or a stereoscopic overlap over a larger region. The further X-ray sources may also be present at a different position in the longitudinal direction, as well as a different position in the lateral direction and/or the vertical direction. This could additionally improve and/or increase the stereoscopic view as there can be a different overlap from different positions. The lateral, longitudinal and vertical directions are defined below.

According to an embodiment of the present invention, a radiation treatment system may incorporate an imaging system as described herein.

A radiation treatment system comprises a treatment system comprising a treatment beam and an imaging system for locating an area of interest within the object. The imaging system according to the present invention can be implemented in such a radiation treatment system to localise the target volume to be treated with radiation. The radiatioan treatment system may include a gantry for delivering a radiation treatment beam. The radiation treatment beam may comprise photons in the MV range. Alternatively, the radiation treatment beam may comprise protons. The radiation treatment system further comprises a patient couch for positioning the patient during the treatment, wherein the couch is the support of the imaging system.

According to an embodiment of the present invention, an imaging system as described herein may be used for motion supervision of the object, in particular during a treatment, a medical intervention or an inspection.

As described above, it is crucial to know the position of a target, for example a tumour or a part of a tumour, within an area of interest such as an organ, a part of an organ or a part of a body such as an arm, a hand, a leg, a foot, the head or the torso. For example, in radiation treatment, the position of the target within the area of interest needs to be determined before and during a treatment or a medical intervention. Due to the geometrical layout of the imaging system according to the present invention, a target can be better localised and the irradiated region can be reduced, when the imaging system is used for motion supervision of the object.

According to an embodiment of the present invention, the imaging system can be equipped with at least one touch guard such as, for example, a proximity sensor and/or a touch sensor. Parts of the imaging system that may be potential collision areas, for example for the object or for surrounding elements, may be equipped with at least one touch guard. Such a touch guard may signal a visual and/or acoustic alarm signal if a predetermined distance, such as for example 10-50 cm, preferably 20-40 cm, is reached. Alternatively, or in addition, the imaging system and/or a surrounding system or element may stop moving until a distance above the predetermined distance is reached.

Useful non-limiting embodiments of the invention are now described with reference to the Figs. Similar elements and/or features are designated with the same reference signs. In the Figs.:
- Fig. 1: shows a schematic cross-sectional view in the lateral direction of a radiation treatment system comprising an imaging system according to an embodiment of the present invention;
- Fig. 2: shows a schematic cross-sectional view in the lateral direction of an imaging system according to an embodiment of the present invention;
- Fig. 3: shows a schematic cross-sectional view in the lateral direction of an imaging system according to an embodiment of the present invention;
- Fig. 4: shows a schematic cross-sectional view in the lateral direction of an imaging system according to an embodiment of the present invention;
- Fig. 5: shows a schematic cross-sectional view in the lateral direction of an imaging system according to an embodiment of the present invention; and
- Fig. 6: shows a schematic cross-sectional view in the longitudinal direction of a radiation treatment system comprising an imaging system according to an embodiment of the present invention.

The figures depict possible exemplary embodiments to illustrate a possibility how the present invention can be implemented. Hereby, not all elements described in relation to the figures are necessary or essential for the exemplary embodiment. Further, exemplary implementations of different embodiments can be combined with each other, as long as the implementations do not technically exclude each other. Similar elements are designated with the same reference signs.

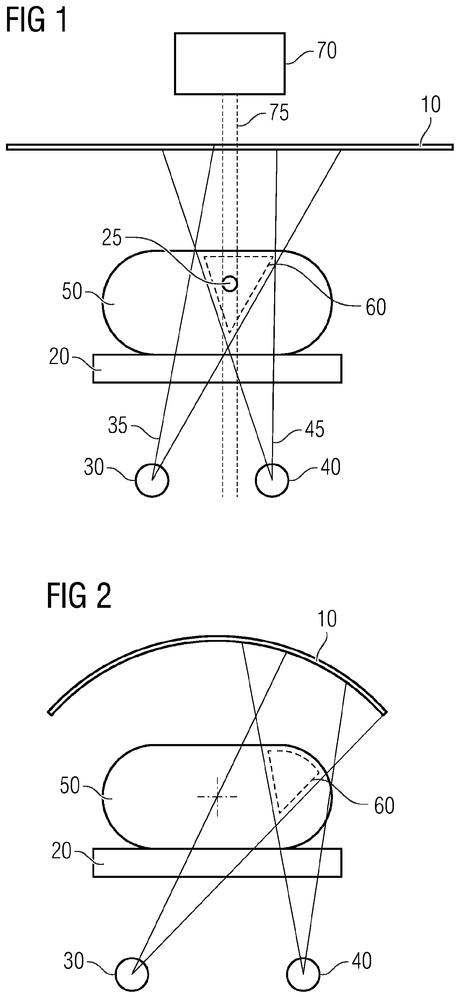

**Figure 1** shows a schematic cross-sectional view in the lateral direction of a radiation treatment system according to an embodiment of the present invention. The radiation treatment system comprises a treatment source 70 and an imaging system according to the present invention. The treatment source 70 is configured to provide a treatment beam 75 that can irradiate a target 25, for example a tumour, within a patient 50 lying on a support 20. The imaging system comprises a first imaging X-ray source 30, a second imaging X-ray source 40 and an imager 10. In this exemplary embodiment, a single flat imager 10 is shown that can receive the radiation of the first and the second imaging X-ray source 30, 40. Such an imager 10 has a substantially planar geometry. The first imaging X-ray source 30 and the second imaging X-ray source 40 are positioned below the support 20 and the imager 10 is positioned above the patient 50 in such a way that an area of interest within the object 50 can be irradiated by the first imaging X-ray source 30 and by the second imaging X-ray source 40. A projection image of the first imaging X-ray source 30 and a projection image of the second imaging X-ray source 40 can be acquired by the imager 10. Hereby, the first imaging X-ray source 30 has a field of view 35 and the second imaging X-ray system 40 has a field of view 45, respectively. As shown in figure 1, the FOV 35 of the first imaging X-ray system 30 and the FOV of the second X-ray system 40 can overlap at least partially. The region where such an overlap appears within the object 50 is the region 60, in which one has stereoscopic view. Thereby, the position of the target 25 can be determined and the treatment beam 75 can be adjusted to cover the target 25 and spare surrounding tissue. It is possible that radiation treatment beam is performed with the treatment beam 75 crossing the imager 10. Alternatively, the imager 10 may be removed prior to the radiation treatment.

The first imaging X-ray source 30, the second imaging X-ray source 40 and the support 20 are configured to move in synchrony with each other.

**Figure 2** shows a schematic cross-sectional view in the lateral direction of an imaging system according to an embodiment of the present invention. The imaging system of figure 2 shows a curved imager 10, while the other elements of the imaging system are the same as in figure 1. Further, regarding a curved imager 10 it is referred to the details described above.

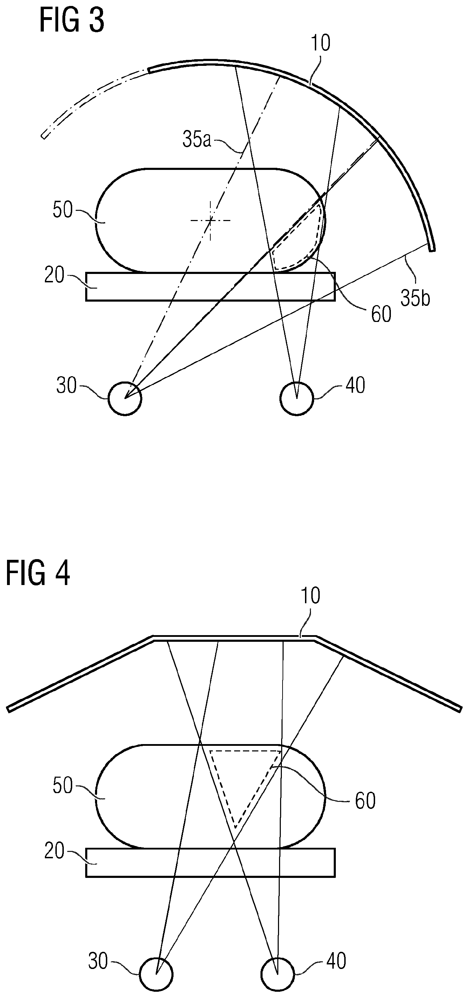

**Figure 3** shows a schematic cross-sectional view in the lateral direction of an imaging system according to an embodiment of the present invention. The imaging system of figure 3 shows a curved imager 10 that is movable as described in detail herein. Hereby, the movement of the imager 10 shown in figure 3 is on a circle segment around the object 50 and there are two exemplary positions shown for said imager 10. The movement can be achieved by a continuous movement or by at least two predetermined steps as described above.

Figure 3 also shows an embodiment where at least one of the imaging X-ray sources 30, 40 is rotatable so that the respective FOV can be adapted as described above. In figure 3, the first imaging X-ray source 30 is depicted with two different field of views 35a, 35b, while the second imaging X-ray source 40 maintains one field of view. The rotational positions of one or both imaging X-ray sources 30, 40 are preferably correlated to the positions of the imager 10, to ensure that the FOVs 35, 45 of the imaging X-ray sources 30, 40 are captured by the imager 10. In the first rotational position, the imager 10 is rotated clockwise, and the X-ray source 30 is rotated to irradiate the field of view 35b. Thereby, the stereo supervision area 60 is in the hatched region. By rotating the imaging X-ray source 30 the position of the stereo supervision area 60 may be moved, as illustrated by the FOV 35a. in order to acquire an image of the new stereo supervision area 60, the imager 10 is rotated anti-clockwise into the second rotational position illustrated in Figure 3. Thereby, the imager may be relatively compact, while at the same time able to acquire images of a large area of interest.

**Figure 4** shows a schematic cross-sectional view in the lateral direction of an imaging system according to an embodiment of the present invention. The imaging system of figure 4 shows a multipart imager 10 comprising three parts. The other elements are the same as in figure 1. Further, the multipart imager 10, the first imaging X-ray source 30 and the second imaging X-ray source 40 can be moved in the same way as described in view of the imager 10 in figure 3. The multipart imager 10 in figure 3 is shown with three parts, wherein two adjacent parts enclose an angle with each other. For a compact design and an extensive imaging system, an angle from 135° to 175° between adjacent parts is preferred, in particular from 150° to 170°.

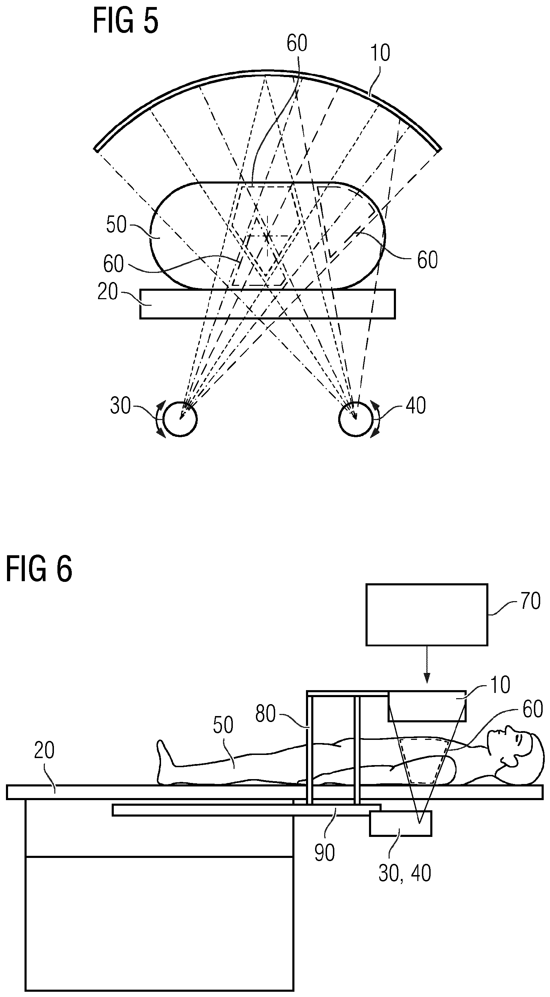

**Figure 5** shows a schematic cross-sectional view in the lateral direction of an imaging system according to an embodiment of the present invention. The imaging system of figure 5 shows a curved imager 10, a rotatable first imaging X-ray source 30 and a rotatable second imaging X-ray source 40. The details of the curved imager 10 and the rotation of imaging X-ray sources 30, 40 are described above. As an example, figure 5 shows three rotating positions for both imaging X-ray sources 30, 40 resulting in three respective field of views for each X-ray source 30, 40. As the relating fields of view of both imaging X-ray sources 30, 40 at least partially overlap, figure 5 shows three exemplary regions 60 of a stereoscopic view within the object 50.

**Figure 6** shows a schematic cross-sectional view in the longitudinal direction of a radiation treatment system according to an embodiment of the present invention. The radiation treatment system comprises a treatment source 70 and an imaging system according to the present invention similar to figure 1.

In addition, the imaging system of figure 6 shows a mounting structure 80 for the imager 10 and a mounting structure 90 for at least one imaging X-ray source 30, 40. Such a respective mounting structure 80, 90 is an example how the imager 10 or at least one imaging X-ray source 30, 40 can be mounted to the support 20. The mounting structure 80 for the imager 10 and the mounting structure 90 for at least one imaging X-ray source 30, 40 may be connected to each other or may only connected to the support 20.

For example, the mounting structure 80, 90 can be a framework that can support the weight of the imager 10 or the imaging X-ray source 30, 40. Such a framework may include rods, plates, nuts and/or bolts.

Each of the mounting structures 80, 90 may be movable in at least one direction, preferably in the longitudinal direction. The mounting structures 80, 90 may be movable together. Each of the mounting structures 80, 90 may be rotatable around at least one axis. Such a movement and/or rotation may be helpful or necessary when the object 50 is placed on the support 20 or to avoid a collision of the imaging system with the object 50 or the treatment source 70.

### List of Reference

- 10: imager
- 20: support
- 25: target
- 30: first imaging X-ray source
- 35: field of view of the first X-ray source
- 35a, 35b: variable field of view of the first X-ray source
- 40: second imaging X-ray source
- 45: field of view of the second X-ray source
- 50: object/patient
- 60: region of a stereoscopic view within the object
- 70: treatment source
- 75: treatment beam
- 80: mounting structure for imager
- 90: mounting structure for imaging X-ray source

## Claims

1. An imaging system for imaging an area of interest of an object (50), comprising:
- a first imaging X-ray source (30),
- a second imaging X-ray source (40),
- at least one imager (10), and
- a moveable support (20), in particular a patient couch, for supporting the object,
wherein the first and second imaging X-ray sources (30, 40) are configured to irradiate overlapping fields of view within the area of interest, such that projection images acquired by the at least one imager (10) result in a stereoscopic view in a region (60) where the fields of view (35, 45) overlap, and
wherein the first imaging X-ray source (30), the second imaging X-ray source (40) and the support (20) are configured to move in synchrony with each other.

2. The imaging system according to claim 1, wherein the at least one imager (10) is configured to move in synchrony with the first imaging X-ray source (30), the second imaging X-ray source (40) and the support (20).

3. The imaging system according to claim 1 or 2, wherein the first imaging X-ray source (30), the second imaging X-ray source (40) and/or the at least one imager (10) are mounted on the support (20).

4. The imaging system according to any of the preceding claims, wherein the first and second imaging X-ray sources are configured to irradiate the same imager (10), wherein the imaging system in particular comprises only a single imager (10).

5. The imaging system according to any of the preceding claims, wherein at least one of the first imaging X-ray source (30) and the second imaging X-ray source (40) is rotatable around its own axis and/or has a rotatable collimation slot, such that the field of view (35, 45) of the at least one imaging X-ray source (30, 40) is rotatable around the axis of the respective imaging X-ray source.

6. The imaging system according to any of the preceding claims, wherein the support (20) has a longitudinal axis, and the at least one imager (10) is curved around said longitudinal axis.

7. The imaging system according to any of the preceding claims, wherein the at least one imager (10) is rotatable and/or movable, preferably on a circle segment, around the object (50), in particular by a predetermined angle and/or by predetermined steps, such that there are at least two positions of the at least one imager (10).

8. The imaging system according to any of the preceding claims, wherein the at least one imager (10) is positioned above the support (20) and the first imaging X-ray source (30) and the second imaging X-ray source (40) are positioned below the support (20) and/or are integrated into the support (20).

9. The imaging system according to any of the preceding claims, wherein the first imaging X-ray source (30) and/or the second imaging X-ray source (40) are movable relative to the support (20), in particular along a longitudinal axis of the support (20).

10. The imaging system according to any of the preceding claims, wherein the first imaging X-ray source (30) and/or the second imaging X-ray source (40) are configured to provide a cone beam.

11. The imaging system according to any of the preceding claims, wherein the at least one imager (10) comprises a scatter rejection grid, in particular a curved scatter rejection grid.

12. The imaging system according to any of the preceding claims, wherein the first imaging X-ray source (30) and/or the second imaging X-ray source (40) are configured to emit multiple X-ray energies and/or wherein the at least one imager (10) comprises at least one energy discrimination multi-layer imager (10) with at least two layers.

13. The imaging system according to any of the preceding claims, wherein the imaging system comprises more than two, preferably between three and six, more preferred three, four or six, imaging X-ray sources (30, 40).

14. A radiation treatment system incorporating an imaging system according to any one of the preceding claims.

15. Use of an imaging system according to any one of the preceding claims 1 to 13 for motion supervision of an object (50), in particular during a treatment, a medical intervention or an inspection.
